# EUROPÄISCHE PATENTSCHRIFT

(11) **EP 3 627 172 B1**
(45) Veröffentlichungstag und Bekanntmachung des Hinweises auf die Patenterteilung: **09.02.2022**
(21) Anmeldenummer: 18195188.0
(22) Anmeldetag: 18.09.2018
(51) Int. Cl.: G01R 33/483, G01R 33/565, G01R 33/561, G01R 33/563

(54) **VERFAHREN UND GERÄT FÜR DAS MRT-SCHICHT-MULTIPLEXING**
METHOD AND APPARATUS FOR SIMULTANEOUS MULTI-SLICE MAGNETIC RESONANCE IMAGING
PROCÉDÉ ET APPAREIL DE MULTIPLEXAGE DE COUCHE EN IMAGERIE PAR RÉSONANCE MAGNÉTIQUE

(43) Veröffentlichungstag der Anmeldung: 25.03.2020
(73) Patentinhaber: Siemens Healthcare GmbH, 91052 Erlangen (DE)
(72) Erfinder: Zeller, Mario, 91054 Erlangen (DE)

(56) Entgegenhaltungen:
- US-A1- 2017 038 450
- US-A1- 2017 089 999
- LYU M. ET AL.: "Robust 2D Nyquist Ghost Correction for Simultaneous Multislice (SMS) EPI Using Phase Error Correction SENSE and Virtual Coil SAKE", PROCEEDINGS OF THE INTERNATIONAL SOCIETY FOR MAGNETIC RESONANCE IN MEDICINE, 25TH ANNUAL MEETING & EXHIBITION, Nr. 515, 7. April 2017 (2017-04-07), XP040688083, Honolulu, HI, USA
- PETERSON E. ET AL.: "Slice-wise Nyquist Ghost Correction for Slice-Accelerated EPI", PROCEEDINGS OF THE INTERNATIONAL SOCIETY FOR MAGNETIC RESONANCE IN MEDICINE, JOINT ANNUAL MEETING ISMRM-ESMRMB, Nr. 1637, 28. April 2014 (2014-04-28), Seite 1637, XP040662706, Milan, Italy
- YARACH U. ET AL.: "Correction of Slice-Specific Nyquist Artifact for Simultaneous Multi-Slice EPI", PROCEEDINGS OF THE INTERNATIONAL SOCIETY FOR MAGNETIC RESONANCE IN MEDICINE, JOINT ANNUAL MEETING ISMRM-ESMRMB, Nr. 4120, 1. Juni 2018 (2018-06-01), XP040703328, Paris, France
- KOOPMANS P.J. ET AL.: "2D-SENSE-GRAPPA For Fast, Ghosting-Robust Reconstruction of In-Plane and Slice Accelerated Blipped-CAIPI-EPI", PROCEEDINGS OF THE INTERNATIONAL SOCIETY FOR MAGNETIC RESONANCE IN MEDICINE, 23RD ANNUAL MEETING & EXHIBITION, Nr. 2410, 15. Mai 2015 (2015-05-15), Seite 2410, XP040668087, Toronto, Canada

## Beschreibung

Verfahren zum Erzeugen von Magnetresonanzbildern, Computerprogramm, Datenträgersignal, computerlesbarer Datenträger und Magnetresonanzanlage

Die Erfindung betrifft ein Verfahren zum Erzeugen von Magnetresonanzbildern eines Zielobjekts und eine hierfür eingerichtete Magnetresonanzanlage. Weitere Aspekte der vorliegenden Erfindung sind ein entsprechendes Computerprogramm oder Computerprogrammprodukt, ein entsprechendes Datenträgersignal und ein computerlesbarer Datenträger, auf welchem ein erfindungsgemäßes Computerprogramm oder Computerprogrammprodukt gespeichert ist.

Die Magnetresonanzbildgebung ist mittlerweile ein etabliertes und wohlbekanntes Fachgebiet, auf welchem zahlreiche unterschiedliche Verfahren zur Datenerfassung und zur Bildgebung bekannt sind. Ein solches Verfahren ist die parallele Bildgebung (PI, englisch: "parallel Imaging"). Eine bekannte Technik der parallelen Bildgebung ist GRAPPA (englisch: "Generalized Autocalibrating Partial Parallel Acquisition"). Bei diesem Verfahren wird nur eine reduzierte Anzahl an Daten aufgenommen und die nicht aufgenommenen Daten werden mit Hilfe von Informationen über die Verteilung mehrerer, um ein jeweiliges Untersuchungsobjekt angeordnete Spulen oder Spulenkanäle rekonstruiert. Bei einem anderen Verfahren können gleichzeitig Daten aus mehreren Schichten (englisch: Slices) eines jeweils abzubildenden Untersuchungs- oder Zielobjekts erfasst werden, was auch als gleichzeitige Mehrschicht- oder Multischicht-Abbildung (SMS, englisch: "Simultaneous Multislice") bezeichnet wird. Bei derartigen Verfahren liegen die Daten der einzelnen gleichzeitig angeregten und abgebildeten Schichten nicht getrennt oder separat voneinander, sondern kollabiert, also einander überlagert vor. Zum Separieren oder Trennen (englisch: uncollapsing) dieser überlagerten Schichtdaten wird bei die sogenannte Schicht-GRAPPA-Technik (englisch: slice GRAPPA), eine Variante des GRAPPA-Verfahrens verwendet. Bei der GRAPPA-Technik werden sogenannte GRAPPA-Kerne (englisch: GRAPPA kernels) zum Rekonstruierten der fehlenden Daten verwendet, wofür eine entsprechende Kalibrierung notwendig ist. Bei der slice- oder Schicht-GRAPPA-Technik werden schichtspezifische GRAPPA-Kerne zum Gewinnen der individuellen Schichten verwendet, wofür ebenfalls eine entsprechende Kalibrierung notwendig ist.

Zum Gewinnen der GRAPPA-Kerne beziehungsweise für die Kalibrierung ebenso wie zur Korrektur von bei der Datenerfassung und/oder einer Erzeugung oder Rekonstruktion von Magnetresonanzbildern aus den erfassten Daten auftretenden Störungen oder Artefakten können ein oder mehrere Vorabscans, auch bezeichnet als Referenzscan oder Korrekturscan, durchgeführt werden. Dieser Vorabscan wird insbesondere vor einem Bildgebungsscan zum Erfassen der eigentlichen Magnetresonanzdaten für die Magnetresonanzbilder des Zielobjekts durchgeführt.

Da bei SMS-Verfahren die einzelnen Schichtdaten kollabiert sind, können nicht ohne Weiteres von nicht-simultanen Abbildungsverfahren bekannte Korrekturmethoden verwendet werden.

Es werden daher bisher verhältnismäßig zeitaufwendige Präparations- und Referenzscans für die Kalibrierung beziehungsweise Artefakt-Korrektur durchgeführt, welche nachteilig einen signifikanten Anteil einer insgesamt zur Untersuchung des Zielobjekts benötigten Zeit beanspruchen können.

Die Veröffentlichung "Motion Robust GRAPPA for Echo-Planar Imaging" von Corey A. Baron und Christian Beaulieu in Magnetic Resonance in Medicine 75:1166-1174 (2016) beschreibt eine Methode zum Akquirieren robuster GRAPPA-Kalibrierungsdaten für eine EPI-basierte Bildgebung (EPI: Echo-Planar Imaging).

Die US 2018 / 0 074 147 A1 beschreibt ein Verfahren zum Implementieren eine TSE-basierten Referenzscans (TSE: Turbo Spin Echo) für die SMS-Akquisition von Magnetresonanzdaten.

Die US 2017 / 0 089 999 A1 befasst sich mit Navigatorbasierten Datenkorrekturen für die SMS-MR-Bildgebung. Konkret sollen dort B₀-Drift-Effekte und N/2-Geist-Effekte in EPI-Daten korrigiert werden. Dabei werden mehrere EPI MR-Datensätze und mehrere Navigatorsequenzen zweier unterschiedlicher Typen generiert. Basierend auf jeweils einer Navigatorsequenz eines ersten und eines zweiten Typs wird wenigstens einer der MR-Datensätze hinsichtlich der unterschiedlichen Effekte korrigiert.

Aus der US 2017 / 0 038 450 A1 sind ein System und Verfahren für die Rekonstruktion Geist-freier Bilder aus Daten bekannt, die mittels SMS-MR-Bildgebung gewonnen wurden. Mit unterschiedlichen Polaritäten der Auslesegradienten erfasste Sätze von Trainingsdaten für verschiedene Schichten eines Patienten werden dabei einem Computerprogramm bereitgestellt, das daraus für jede Schicht Geist-freie Bilder erzeugt. Ausgehend von einer jeweiligen Kombination der Trainingsdatensätze werden positive und negative synthetische kollabierte Auslesedaten entsprechend einer simultanen Akquisition erzeugt. Weiter wird für jede Schichtposition ein Rekonstruktionskern berechnet mittels eines Trainingsalgorithmus, der die kollabierten Auslesedaten als Quelldaten und die Geist-freien Bilder als Zieldaten verwendet. Durch Anwenden der Rekonstruktionskerne werden dann für die Schichten Bilder rekonstruiert, die im Wesentlichen frei von Geist-Artefakten sind.

Aufgabe der vorliegenden Erfindung ist es, eine beschleunigte Datenakquisition für Magnetresonanzbilder mittels eines PI-Verfahrens (PI: Parallel Imaging, parallele Bildgebung) zu ermöglichen. Diese Aufgabe wird erfindungsgemäß durch die Gegenstände der unabhängigen Patentansprüche gelöst. Vorteilhafte Ausgestaltungen und Weiterbildungen der vorliegenden Erfindung sind in den abhängigen Patentansprüchen, in der Beschreibung und in den Zeichnungen angegeben.

Ein erfindungsgemäßes Verfahren dient zum Erzeugen von Magnetresonanzbildern eines Zielobjekts. Das Ziel- oder Untersuchungsobjekt kann beispielsweise ein Patient, ein Gewebebereich oder Organ eines Patienten, eine Gewebeprobe oder ein anderes der Magnetresonanzbildgebung zugängliches Objekt sein.

In einem Verfahrensschritt des erfindungsgemäßen Verfahrens wird für jede abzubildende Schicht des Zielobjekts ein separater Referenzdatensatz mittels einer Magnetresonanzanlage aufgenommen durch Ausführen eines jeweiligen Referenzscans des Zielobjekts für jede abzubildende Schicht des Zielobjekts mittels einer GRE-Sequenz (GRE: Gradienten-Echo) oder mittels einer RARE-Sequenz (RARE: Rapid Acquisition with Refocused Echoes). Die RARE-Sequenz kann beispielsweise eine TSE- oder eine HAESTE-Sequenz sein (HASTE: Half fourier-Acquired Single shot Turbo spin Echo). Mittels der GRE-Sequenz kann der Referenzscan vorteilhaft typischerweise besonders schnell ausgeführt werden, da nur ein Hoch- oder Radiofrequenz-Puls (RF-Puls) appliziert oder verwendet und somit ein Echo besonders schnell, also nach besonders kurzer Zeit aufgezeichnet, also erfasst werden kann. Die RARE-Sequenz kann hingegen vorteilhaft beispielsweise bei besonders großen Variationen des B₀-Magnetfeldes besonders gute Ergebnisse liefern.

In einem weiteren Verfahrensschritt des erfindungsgemäßen Verfahrens wird für jede abzubildende Schicht des Zielobjekts ein separater Korrekturdatensatzes mittels der Magnetresonanzanlage aufgenommen durch Ausführen eines jeweiligen Phasenkorrekturscans des Zielobjekts für jede abzubildende Schicht des Zielobjekts mittels einer nicht-phasenkodierenden oder nicht-phasenkodierten EPI-Sequenz. Dies kann auch als Navigator-Daten oder Erfassen von Navigator-Echos bezeichnet werden.

In einem weiteren Verfahrensschritt des erfindungsgemäßen Verfahrens wird ein Messdatensatz des Zielobjekts mittels der Magnetresonanzanlage aufgenommen mittels einer SMS-Sequenz.

Die SMS-Sequenz dient also als Bildgebungsscan zum Erfassen oder Aufnehmen von Magnetresonanzdaten oder Rohdaten des Zielobjekts, aus denen die Magnetresonanzbilder der abgebildeten Schichten des Zielobjekts erzeugt oder rekonstruiert werden.

In einem weiteren Verfahrensschritt des erfindungsgemäßen Verfahrens werden aus dem Referenzdatensatz schichtspezifische GRAPPA-Kerne bestimmt. Es wird also für jede abgebildete Schicht des Zielobjekts wenigstens ein GRAPPA-Kern bestimmt oder berechnet.

In einem weiteren Verfahrensschritt des erfindungsgemäßen Verfahrens werden mittels einer entsprechend eingerichteten Datenverarbeitungseinrichtung die Magnetresonanzbilder des Zielobjekts aus dem Messdatensatz erzeugt oder rekonstruiert durch ein Schicht-GRAPPA-Verfahren (englisch: slice GRAPPA). Zu unterschiedlichen der abgebildeten Schichten gehörende Daten des Messdatensatzes werden dabei mittels der schichtspezifischen GRAPPA-Kerne voneinander separiert oder getrennt (englisch: uncollapsed). Nyquist-N/2-Geist-Artefakte, im Folgenden kurz als N/2-Geist-Artefakte oder Geist-Artefakte bezeichnet, werden in dem Messdatensatz oder in den Magnetresonanzbildern dabei mittels des Korrekturdatensatzes oder basierend auf dem Korrekturdatensatz korrigiert.

Es wird vorliegend also vorgeschlagen, einen dedizierten EPI-basierten Phasenkorrekturscan zur N/2-Geist-Korrektur und zusätzlich den GRE- oder RARE-basierten Referenzscan zum Akquirieren von Referenz- und Korrekturdaten für ein paralleles Bildgebungsverfahren (PI-Verfahren) zu verwenden. Dadurch kann auf einen herkömmlicherweise vorgesehenen, deutlich längeren EPI-basierten Referenzscan für das Schicht-GRAPPA-Verfahren verzichtet werden. Dies führt dazu, dass durch das erfindungsgemäße Verfahren die Referenz- und Korrekturdaten im Gegensatz zu der herkömmlichen EPI-basierten Methode schneller erfasst oder akquiriert werden können. Mittels der vorliegenden Erfindung kann vorteilhaft also eine verkürzte Gesamtuntersuchungszeit erreicht werden. Die vorliegende Erfindung basiert dabei auf der Erkenntnis, dass ein GRE- oder RARE-basierter Referenzscan zum Akquirieren von Referenzdaten für die Schicht-GRAPPA-Kalibrierung verwendet werden kann und dann fehlende Daten für die Korrektur der Geist-Artefakte mittels des hier vorgeschlagenen Phasenkorrektur- oder Navigator-Scans gewonnen werden können.

In einem herkömmlichen Verfahren würden beispielsweise ein GRE-basierter Referenzscan von beispielsweise 3 s Länge für eine in-plane GRAPPA-Kalibrierung sowie ein dummy EPI-Scan und ein EPI-Referenzscan für die Schicht-GRAPPA-Kalibrierung von zusätzlich beispielsweise 10 s Länge für 20 Schichten durchgeführt werden, sodass also vor einem eigentlichen Bildgebungsscan bereits wenigstens 13 s für die Vorabscans benötigt werden. Insbesondere für SMS-beschleunigte diffusionsbasierte Sequenzen mit einer Gesamtmesszeit von beispielsweise nur etwa einer Minute führt dies zu einem signifikanten Zeitverlust, wodurch die Vorteile der SMS-basierten Bildgebung zumindest teilweise wieder zunichte gemacht werden. Demgegenüber benötigen die als Teil der vorliegenden Erfindung vorgesehenen Vorabscans deutlich weniger Zeit, nämlich beispielsweise die gleichen 3 s für den GRE-basierten Referenzscan und beispielsweise 100 ms bis 200 ms für den Phasenkorrekturscan.

Der Phasenkorrekturscan kann vorliegend vorteilhaft als vergleichsweise schneller Einzelband (englisch: single-band) Scan durchgeführt werden. Der Messdatensatz kann hingegen für eine besonders genaue und detailreiche Abbildung mittels eines Multiband Bildgebungsscans erfasst werden.

Für das Trennen der Daten der abgebildeten Schichten mittels der schichtspezifischen GRAPPA-Kerne und für das Korrigieren der Nyquist-N/2-Geist-Artefakte können auf dem Fachgebiet an sich bekannte Methoden verwendet werden.

Die Verfahrensschritte des erfindungsgemäßen Verfahrens können in der beschriebenen Reihenfolge aber ebenso auch in anderen Reihenfolgen ausgeführt werden. Es kann beispielsweise möglich sein, den Messdatensatz vor dem Referenzdatensatz und/oder dem Korrekturdatensatz aufzunehmen.

In vorteilhafter Ausgestaltung der vorliegenden Erfindung werden die Referenzscans für alle Schichten aufeinanderfolgend ausgeführt und die Phasenkorrekturscans für alle Schichten ebenfalls aufeinanderfolgend ausgeführt. Dabei werden alle Phasenkorrekturscans ausgeführt nachdem alle der Referenzscans ausgeführt worden sind oder bevor alle der Referenzscans ausgeführt werden. Mit anderen Worten werden also jeweils gebündelt zunächst alle Referenzscans und danach alle Phasenkorrekturscans ausgeführt oder umgekehrt. Diese Varianten des Verfahrens sind vorteilhaft besonders einfach zu implementieren, da entsprechende Blöcke oder Bausteine für eine Gesamtsequenz bereits in herkömmlichen Datensammlungen oder Datenbanken zur Sequenzerzeugung verfügbar sind.

In einer alternativen vorteilhaften Ausgestaltung der vorliegenden Erfindung werden die Referenzscans und die Phasenkorrekturscans in verschachtelter oder miteinander verschränkter Reihenfolge ausgeführt. Dabei werden für jede der abzubildenden Schichten der jeweilige Referenzscan und der jeweilige Phasenkorrekturscan ausgeführt, bevor die jeweiligen Referenz- und Phasenkorrekturscans für die jeweils nächste der abzubildenden Schichten ausgeführt werden. Für jede einzelne der Schichten kann dabei zuerst der Referenzscan und dann der Phasenkorrekturscan ausgeführt werden. Ebenso kann umgekehrt für jede einzelne der abzubildenden Schichten zunächst der Phasenkorrekturscan und dann der Referenzscan ausgeführt werden. Die Referenz- und Phasenkorrekturscans werden also beispielsweise abwechselnd ausgeführt, während die abzubildenden Schichten der Reihe nach gescannt werden. Diese verschachtelte, verschränkte oder versetzte Ausführungsreihenfolge der Referenz- und Phasenkorrekturscans wird auch als Interleaving bezeichnet. Da somit also für jede Schicht der jeweilige Referenzscan und der jeweilige Phasenkorrekturscan unmittelbar aufeinanderfolgend ausgeführt werden, liegen für beide Vorabscans für eine bestimmte Schicht zumindest im Wesentlichen dieselben steady-states, also Gleichgewichtszustände oder Gleichgewichtseffekte vor und beide Vorabscans für eine bestimmte Schicht werden zumindest im Wesentlichen in derselben Atem- oder Bewegungsphase des Zielobjekts, also mit minimaler räumlicher Verschiebung des Zielobjekts zwischen den beiden Vorabscans für eine Schicht ausgeführt. Hierdurch kann gegebenenfalls ein Auftreten von Bewegungsartefakten vermieden oder reduziert werden.

In vorteilhafter Weiterbildung der vorliegenden Erfindung wird bei dem Aufnehmen der Vorabscans, also des Referenzscans und des Phasenkorrekturscans, für jeweils eine der Schichten nur ein Anregungspuls erzeugt und nach diesem der jeweilige Referenzscan und der jeweilige Phasenkorrekturscan für die jeweilige Schicht ausgeführt, ohne dass zwischen dem Referenzscan und dem Phasenkorrekturscan für die jeweilige Schicht ein zweiter Anregungspuls für diese Schicht erzeugt oder angewendet wird. Mit anderen Worten wird also für eine Schicht ein Anregungspuls erzeugt, dann der Referenzscan oder der Phasenkorrekturscan für diese Schicht ausgeführt und eine verbleibende Magnetisierung der Schicht dann für den zweiten Vorabscan dieser Schicht, also für den Phasenkorrekturscan beziehungsweise den Referenzscan, genutzt. Hierdurch können die beiden Vorabscans ohne Verzögerung unmittelbar nacheinander ausgeführt werden, sodass die benötigte Akquisitionszeit und damit auch die für die Untersuchung des Zielobjekts insgesamt benötigte Zeit weiter reduziert, also die Datenerfassung weiter beschleunigt werden kann. Das Ausnutzen der durch einen einzigen Anregungspuls erzeugten Magnetisierung für den jeweiligen Referenz- und Phasenkorrekturscan ist hier relativ einfach realisierbar, da der Phasenkorrekturscan für eine Schicht deutlich schneller ausgeführt werden kann, als der Referenzscan. Beispielsweise kann der Phasenkorrekturscan für eine Schicht in ungefähr 5 ms ausgeführt werden, sodass hierfür beziehungsweise zum Ausführen des Referenzscans nach dem einen Anregungspuls noch ausreichend Magnetisierung in der jeweiligen Schicht vorliegt, also gegeben ist.

Bei dem Phasenkorrekturscan werden für jeweils eine der abzubildenden Schichten wenigstens zwei Echos erfasst oder aufgezeichnet. In besonders vorteilhafter Ausgestaltung der vorliegenden Erfindung werden für den Korrekturdatensatz aber jeweils wenigstens drei Echos aus jedem der Phasenkorrekturscans verwendet. Dabei wird ein Mittelwert aus dem ersten und dem dritten Echo gebildet, um eine zeitliche Konsistenz mit dem jeweiligen zweiten Echo zu erreichen. Dies kann entsprechend übertragen für weitere Echos angewendet werden, falls mehr als drei Echos akquiriert werden. Auf diese Weise kann vorteilhaft eine besonders genaue Phasen- oder Geist-Artefakt-Korrektur erreicht werden, da eine während des Phasenkorrekturscans zwischen den zeitlich nacheinander erfassten Echos auftretende Phasenverschiebung berücksichtigt wird. Besonders bevorzugt können ebenso mehr als drei Echos, beispielsweise 10 Echos aus jedem Phasenkorrekturscan, also für jede der abzubildenden Schichten erfasst werden. Eine größere Anzahl von bei den einzelnen Phasenkorrekturscans erfassten Echos kann dabei zu einer verbesserten Genauigkeit oder Zuverlässigkeit der Phasenkorrektur beziehungsweise der Korrektur der Geist-Artefakte beitragen. Besonders bevorzugt können dabei ebenso Wirbelstromeffekte berücksichtigt werden, wodurch die Genauigkeit der Korrektur weiter verbessert werden kann.

In weiterer vorteilhafter Ausgestaltung der vorliegenden Erfindung werden für die Referenzscans und die Phasenkorrekturscans Flipwinkel, also Anregungswinkel, von weniger als 90° verwendet. Mit anderen Worten werden also schwächere oder kürzere Anregungspulse für die Vorabscans verwendet als beispielsweise für den Bildgebungsscan, also die SMS-Sequenz zum Aufnehmen des Messdatensatzes. Auf diese Weise können vorteilhaft Sättigungs- oder Vor-Sättigungseffekte vor Beginn des Bildgebungsscans vermieden oder reduziert werden. Da der Referenzdatensatz und der Korrekturdatensatz zwar bei dem Erzeugen oder Rekonstruieren der Magnetresonanzbilder verwendet werden, jedoch nicht unmittelbar in den Magnetresonanzbildern dargestellte Daten enthalten, können die Flipwinkel von weniger als 90°, also die entsprechend schwächeren oder kürzeren Anregungspulse verwendet werden, ohne dass hierdurch beispielsweise ein Kontrast der erzeugten Magnetresonanzbilder negativ beeinträchtigt, also verringert würde.

In weiterer vorteilhafter Ausgestaltung der vorliegenden Erfindung wird der Messdatensatz mittels einer multiband SMS-EPI-Sequenz aufgenommen, insbesondere für eine diffusionsbasierte Abbildung des Zielobjekts. Eine diffusionsbasierte Abbildung des Zielobjekts kann beispielsweise eine diffusionsgewichtete Bildgebung (DWI, englisch: diffusion-weighted imaging), eine Diffusions-Spektrum-Abbildung (DSI, englisch: diffusion spectrum imaging) oder eine Diffusions-Tensor-Abbildung (DTI, englisch: diffusion tensor imaging) sein oder umfassen. Dieser Ausgestaltung der vorliegenden Erfindung liegt die Erkenntnis zugrunde, dass dies ein Fall ist, in dem die vorliegende Erfindung besonders vorteilhaft und nutzbringend angewendet werden kann. Dies liegt darin begründet, dass hier eine durch die vorliegende Erfindung gegenüber herkömmlichen Verfahren beziehungsweise Referenzscans erreichte Zeitersparnis einen besonders großen prozentualen Anteil an einer für die Untersuchung des Zielobjekts insgesamt benötigten Zeitdauer ausmacht. Bei einer SMS-beschleunigten Diffusionssequenz mit einer Gesamtmesszeit von ungefähr einer Minute können durch die vorliegende Erfindung beispielsweise bis zu 15 % der Gesamtmesszeit eingespart werden gegenüber einem herkömmlichen Verfahren, bei dem ein zusätzlicher EPIbasierter dummy- und Referenzscan für das Schicht-GRAPPA-Verfahren durchgeführt wird.

In weiterer vorteilhafter Ausgestaltung der vorliegenden Erfindung wird der Referenzdatensatz mit geringerer Auflösung aufgenommen als der Messdatensatz. Werden für eine Schicht oder ein Segment einer Schicht beispielsweise 256 Zeilen für den Messdatensatz aufgenommen oder abgetastet, so können für dieselbe Schicht beziehungsweise dasselbe Segment für den Referenzdatensatz beispielsweise 8, 16 oder 32 Zeilen aufgenommen oder abgetastet werden. Diese reduzierte Auflösung des Referenzdatensatzes ermöglicht vorteilhaft eine beschleunigte Datenakquisition und somit eine reduzierte Gesamtmesszeit für die Untersuchung des Zielobjekts.

In weiterer vorteilhafter Ausgestaltung der vorliegenden Erfindung werden Sensitivitätsvariationen zwischen verschiedenen für das Aufnehmen des Messdatensatzes parallel verwendeten Empfängerspulen bestimmt und bei dem Erzeugen der Magnetresonanzbilder berücksichtigt. Die Empfängerspulen können dabei insbesondere auf oder an dem Zielobjekt angeordnet sein, um die parallele Bildgebung zu ermöglichen. Die Sensitivitätsvariationen, also spulenspezifische Eigenschaften oder Parameter der einzelnen Empfängerspulen, können also ausgewertet und berücksichtigt werden, um eine verbesserte Genauigkeit beim Erzeugen der Magnetresonanzbilder zu erreichen. Mittels der einzelnen Empfängerspulen erfasste oder gemessene Signale oder den einzelnen Empfängerspulen zugeordnete Datenströme, werden dabei auch als Spulenkanäle bezeichnet.

Die Sensitivitätsvariationen, also die Spulendaten oder Spuleneigenschaften der einzelnen Empfängerspulen, können insbesondere in Kombination mit einer gegenüber dem Messdatensatz reduzierten Auflösung des Referenzdatensatzes vorteilhaft ausgenutzt werden. Nachdem alle Daten aufgenommen oder erfasst worden sind, ist grundsätzlich bekannt, wie die einzelnen Schichten aussehen müssen, sodass auch bei reduzierter Auflösung des Referenzdatensatzes insbesondere unter Berücksichtigung der Sensitivitätsvariationen die Schicht-GRAPPA-Kalibrierung beziehungsweise das Trennen der zu den unterschiedlichen Schichten gehörenden Daten oder Datenteile des Messdatensatzes durchgeführt werden kann. Effektiv kann eine Multibandakquisition simuliert werden auch wenn der Referenzscan als Einzelband-Scan ausgeführt wurde.

In weiterer vorteilhafter Ausgestaltung der vorliegenden Erfindung wird zusätzlich zu dem Schicht-GRAPPA-Verfahren ein in-plane-GRAPPA-Verfahren, also eine in-plane-Beschleunigung, verwendet oder angewendet. Der Referenzdatensatz wird dann auch zum Kalibrieren der GRAPPA-Kerne für das in-plane-GRAPPA-Verfahren verwendet. Mit anderen Worten kann der Referenzscan beziehungsweise der Referenzdatensatz also sowohl für die slice- oder Schicht-GRAPPA-Kalibrierung als auch für die in-plane GRAPPA-Kalibrierung verwendet werden. Es können auf diese Weise also vorteilhaft das Schicht-GRAPPA-Verfahren und das in-plane GRAPPA-Verfahren miteinander kombiniert und dabei nur ein einziger Referenzscan für jede der abzubildenden Schichten ausgeführt werden. Somit kann eine Geschwindigkeit und Effizienz des erfindungsgemäßen Verfahrens weiter verbessert werden.

Ein weiterer Aspekt der vorliegenden Erfindung ist ein Computerprogramm oder Computerprogrammprodukt, welches Befehle, Programmmodule oder Steueranweisungen umfasst, die bei einer Ausführung des Computerprogramms oder Computerprogrammprodukts durch eine Computereinrichtung einer Magnetresonanzanlage die Magnetresonanzanlage dazu veranlassen, das erfindungsgemässe Verfahren bzw. eine Ausführungsform dieses Verfahrens zum Erzeugen von Magnetresonanzbildern eines Zielobjekts, insbesondere automatisch, auszuführen. Die Computereinrichtung kann dabei ein Computer, ein Steuergerät, eine Datenverarbeitungseinrichtung oder dergleichen sein oder umfassen. Insbesondere kann die Computereinrichtung eine Prozessoreinrichtung, also einen Prozessor, einen Mikrochip oder einen Mikrocontroller, zum Ausführen des Computerprogramms oder Computerprogrammprodukts aufweisen. Das erfindungsgemäße Computerprogramm oder Computerprogrammprodukt kodiert oder repräsentiert also die Anweisungen, Vorgänge oder Verfahrensschritte des erfindungsgemäßen Verfahrens. Insbesondere ist das erfindungsgemäße Computerprogramm oder Computerprogrammprodukt zum Laden in einen, insbesondere elektronischen und/oder elektronisch lesbaren, insbesondere computerlesbaren, Datenspeicher oder Datenträger einer Magnetresonanzanlage oder einer Datenverarbeitungseinrichtung zum Ausführen der Verfahrensschritte eingerichtet.

Ein weiterer Aspekt der vorliegenden Erfindung ist ein Datenträgersignal, welches das erfindungsgemässe Computerprogramm überträgt. Das erfindungsgemäße Datenträgersignal kann also beispielsweise von einem elektronisch lesbaren oder computerlesbaren Datenträger zu einer Magnetresonanzanlage oder einer Datenverarbeitungseinrichtung zum Ausführen des erfindungsgemäßen Verfahrens übertragen werden. Diese Übertragung kann beispielsweise über eine kabelgebundene oder kabellose Datenverbindung erfolgen. Ein weiterer Aspekt der vorliegenden Erfindung ist ein computerlesbarer, also insbesondere elektronisch lesbarer, Datenträger oder Datenspeicher, auf oder in welchem zumindest eine Ausführungsform des erfindungsgemäßen Computerprogramms oder Computerprogrammprodukts gespeichert ist. Der erfindungsgemäße Datenträger kann insbesondere ein Datenträger oder Datenspeicher für eine Datenverarbeitungseinrichtung und/oder ein Steuergerät einer Magnetresonanzanlage oder eine mit einer Magnetresonanzanlage verbundene Datenverarbeitungseinrichtung sein. In oder auf dem erfindungsgemäßen Datenträger können, insbesondere als Teil des dort gespeicherten erfindungsgemäßen Computerprogramms, ein Programmcode und/oder weitere Steueranweisungen für die Datenverarbeitungseinrichtung oder die Magnetresonanzanlage gespeichert oder kodiert sein.

Ein weiterer Aspekt der vorliegenden Erfindung ist eine Magnetresonanzanlage mit einer Erfassungs- oder Aufnahmeeinrichtung zum Aufnehmen von Magnetresonanzdaten und mit einer Datenverarbeitungseinrichtung zum Verarbeiten der aufgenommenen Magnetresonanzdaten. Die Datenverarbeitungseinrichtung umfasst dabei einen erfindungsgemäßen computerlesbaren Datenträger und eine mit diesem verbundene Prozessoreinrichtung, also insbesondere einen Prozessor, einen Mikrochip oder einen Mikrocontroller, welche zum Ausführen des auf dem computerlesbaren Datenträger gespeicherten erfindungsgemäßen Computerprogramms oder Computerprogrammprodukts, also Programmcodes, zum Ausführen zumindest einer Ausführungsform des erfindungsgemäßen Verfahrens eingerichtet ist. Die erfindungsgemäße Magnetresonanzanlage ist also dazu eingerichtet, mittels der Aufnahmeeinrichtung einen Referenzdatensatz aufzunehmen durch Ausführen eines Referenzscans des Zielobjekts für jede abzubildende Schicht des Zielobjekts mittels einer GRE-Sequenz oder einer RARE-Sequenz. Weiter ist die erfindungsgemäße Magnetresonanzanlage dazu eingerichtet, mittels der Aufnahmeeinrichtung einen Korrekturdatensatz durch Ausführen eines Phasenkorrekturscans des Zielobjekts für jede abzubildende Schicht des Zielobjekts mittels einer nicht-phasenkodierenden oder nicht-phasenkodierten EPI-Sequenz aufzunehmen. Weiter ist die erfindungsgemäße Magnetresonanzanlage dazu eingerichtet, mittels der Aufnahmeeinrichtung einen Messdatensatz des Zielobjekts mittels einer SMS-Sequenz aufzunehmen. Weiter ist die erfindungsgemäße Magnetresonanzanlage dazu eingerichtet, mittels der Datenverarbeitungseinrichtung, insbesondere automatisch, die aufgenommenen Magnetresonanzdaten, also den Referenzdatensatz, den Korrekturdatensatz und den Messdatensatz, zu verarbeiten und dabei aus dem Referenzdatensatz schichtspezifische GRAPPA-Kerne für die einzelnen abgebildeten Schichten zu bestimmen und die Magnetresonanzbilder des Zielobjekts aus dem Messdatensatz durch ein Schicht-GRAPPA-Verfahren zu erzeugen. Die Datenverarbeitungseinrichtung ist dazu eingerichtet, dabei zu unterschiedlichen der abgebildeten Schichten gehörende Daten des Messdatensatzes mittels der schichtspezifischen GRAPPA-Kerne voneinander zu separieren oder zu trennen und Nyquist-N/2-Geist-Artefakte in dem Messdatensatz oder in den Magnetresonanzbildern mittels des Korrekturdatensatzes oder basierend auf den Korrekturdatensatz zu korrigieren.

Die erfindungsgemäße Magnetresonanzanlage kann also insbesondere die im Zusammenhang mit dem erfindungsgemäßen Verfahren und/oder im Zusammenhang mit den anderen Aspekten der vorliegenden Erfindung genannte Magnetresonanzanlage sein. Dementsprechend kann die erfindungsgemäße Magnetresonanzanlage einige oder alle der im Zusammenhang mit dem erfindungsgemäßen Verfahren und/oder den anderen Aspekten der vorliegenden Erfindung genannten Eigenschaften und/oder Bauteile oder Komponenten aufweisen.

Die bisher und im Folgenden angegebenen Eigenschaften und Weiterbildungen des erfindungsgemäßen Verfahrens, der erfindungsgemäßen Magnetresonanzanlage und der übrigen Aspekte der vorliegenden Erfindung sowie die entsprechenden Vorteile sind jeweils sinngemäß wechselseitig zwischen dem erfindungsgemäßen Verfahren, der erfindungsgemäßen Magnetresonanzanlage und den übrigen Aspekte der vorliegenden Erfindung übertragbar. Es gehören also zu der Erfindung also auch solche Weiterbildungen der Aspekte der vorliegenden Erfindung, welche Ausgestaltungen aufweisen, die hier zur Vermeidung unnötiger Redundanz nicht explizit in der jeweiligen Kombination oder für jeden Aspekt der Erfindung separat beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:
- FIG 1: eine schematische Illustration einer herkömmlichen nicht-simultanen Mehrschicht-Datenerfassung in der Magnetresonanzbildgebung;
- FIG 2: eine schematische Illustration eines Sequenzdiagramms für eine herkömmlichen SMS-EPI-Sequenz;
- FIG 3: eine schematische ausschnittweise Illustration eines Sequenzdiagramms einer verbesserten Sequenz für eine SMS-Magnetresonanzbildgebung in einer ersten Ausführungsform;
- FIG 4: eine schematische ausschnittweise Illustration eines Sequenzdiagramms einer verbesserten Sequenz für eine SMS-Magnetresonanzbildgebung in einer zweiten Ausführungsform; und
- FIG 5: eine schematische Darstellung einer Magnetresonanzanlage.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den Figuren sind gleiche, funktionsgleiche oder einander entsprechende Elemente jeweils mit den gleichen Bezugszeichen gekennzeichnet.

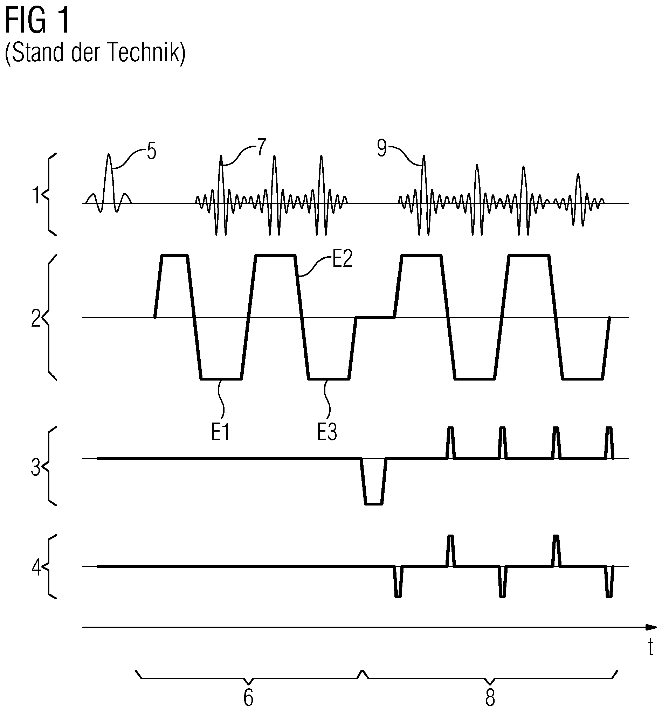

Bekannte Multischicht EPI-Sequenzen, bei denen die einzelnen Schichten nacheinander, also nicht-simultanen, angeregt und abgetastet werden, erfassen typischerweise ein Navigator-Echo bevor die eigentlichen Bilddaten für jede Schicht erfasst werden. FIG 1 zeigt hierzu eine schematische Illustration einer solchen herkömmlichen nicht-simultanen mehr Schicht-Datenerfassung in der Magnetresonanzbildgebung. Es sind hier parallel zueinander vier Signale über der Zeit t aufgetragen. Als erstes Signal 1 ist hier ein RF- beziehungsweise ADC-Signal (ADC: apparent diffusion coefficient) aufgetragen. Als zweites Signal 2 ist ein zugehöriger Readout-Gradient, also ein Auslesegradient, aufgetragen. Als drittes Signal 3 ist ein zugehöriger Phasenkodierungsgradient und als viertes Signal 4 ein zugehöriger Schichtgradient aufgetragen.

Zu einem hier dargestellten Beginn einer Gesamtsequenz wird ein Anregungspuls 5 eingestrahlt. In einem sich daran anschließenden Zeitabschnitt 6 wird eine Navigatorsequenz 7 aus drei nicht-phasenkodierten Navigator-Echos ausgeführt beziehungsweise akquiriert. Dazu werden drei nicht-phasenkodierte Auslesegradienten E1, E2, E3 erzeugt, wobei die Auslesegradienten E1 und E3 negative Auslesegradientenpolaritäten (englisch: readout gradient polarities) und der Auslesegradient E2 eine positive Auslesegradientenpolarität aufweisen. Entsprechend werden die zu den Auslesegradienten E1, E3 korrespondierenden Navigator-Echos auch als ungerade Echos und das zu dem Auslesegradienten E2 korrespondierende Navigator-Echo als gerades Echo bezeichnet(englisch: odd and even echoes).

In einem sich an den Zeitabschnitt 6 anschließenden Zeitabschnitt 8 wird dann eine eigentliche Bildgebungssequenz 9 ausgeführt, um Magnetresonanzdaten zu akquirieren, aus denen dann Magnetresonanzbilder der einzelnen Schichten erzeugt oder rekonstruiert werden können.

Die Navigatorsequenz 7 kann während der Rekonstruktion der Magnetresonanzbilder typischerweise für zwei unterschiedliche Korrekturen verwendet werden, nämlich für eine schichtspezifische Nyquist-N/2-Geist-Artefakt-Korrektur einerseits und eine B₀-Feld-Drift-Korrektur andererseits. Für die Geist-Artefakt-Korrektur werden Phasenverschiebungen nullter und erster Ordnung zwischen ungeraden und geraden Navigator-Echos beziehungsweise Auslesegradienten E1, E3, E2 in Ausleserichtung bestimmt, indem entsprechende Daten aus dem Bildraum miteinander korreliert werden. Damit können während der Rekonstruktion der Magnetresonanzbilder nachträglich oder rückwirkend ungerade und gerade k-Raum-Linien neu ausgerichtet werden. Dadurch können Verschiebungen kompensiert werden, welche beispielsweise durch Faktoren wie Gradientenverzögerungen (englisch: gradient delays) oder Wirbelströme verursacht werden. Für die B₀-Feld-Drift-Korrektur kann die an sich bekannte DORK-Methode (englisch: Dynamic Off-Resonance in k-space) verwendet werden. Die zeitliche Drift des B₀-Feldes wird dabei bestimmt durch Vergleich einer Phasenevolution der Navigator-Echos beziehungsweise der Auslesegradienten E1, E3 beziehungsweise E2 mit identischen Auslesegradientenpolaritäten zwischen nacheinander erfassten Bildvolumen.

Entsprechende Berechnungen werden dabei typischerweise als Durchschnitt über ein vollständiges Bildvolumen ausgeführt.

In der simultanen Mehrschicht-EPI (SMS-EPI) werden hingegen mehrere Schichten gleichzeitig angeregt und entsprechende Magnetresonanzdaten für diese mehreren angeregten Schichten werden gleichzeitig erfasst. Dies führt zu einem k-Raum Datensatz, welcher einander überlagerte Daten von oder aus mehreren Schichten, also mehrere aufeinander kollabierte Schichten umfasst. Eine Separierung oder Trennung dieser Schichten beziehungsweise der zu unterschiedlichen Schichten gehörenden Daten wird während der Rekonstruktion der Magnetresonanzbilder mittels eines Schicht-GRAPPA-Verfahrens (englisch: slice GRAPPA method) durchgeführt. Das Schicht-GRAPPA-Verfahren trennt die einzelnen Schichten beziehungsweise die schichtspezifischen Daten mittels jeweiliger schichtspezifischer GRAPPA-Kerne (englisch: slice-specific GRAPPA kernels). Diese Kerne können aus einem separaten nicht-kollabierten Vorabscan vor dem Erfassen der kollabierten, also einander überlagerten Mess- oder Bildgebungs-, also Magnetresonanzdaten, gewonnen werden.

Für SMS-Akquisitionen sind jedoch die mittels der Navigatorsequenz 7 erfassten Navigator-Echos beziehungsweise entsprechende Navigator-Daten ebenfalls kollabiert, also einander überlagert. Die DORK-Methode führt Durchschnittsberechnungen über das gesamte Bildvolumen durch, weswegen die kollabierten Navigator-Daten direkt für diese Methode, also die B₀-Feld-Drift-Korrektur verwendet werden können. Die kollabierten Navigator-Daten können jedoch nicht für die schichtspezifische Geist-Artefakt-Korrektur verwendet werden, da es bislang keine bekannte Methode gibt, die nicht-phasenkodierten Navigator-Daten zu trennen, also zu dekollabieren.

Wenn eine in-plane-Beschleunigung, also ein In-plane GRAPPA-Verfahren zusätzlich zu der simultanen Mehrschicht-Abbildung (SMS, englisch: Simultaneous Multislice) verwendet wird, wird herkömmlicherweise ein in-plane GRAPPA Referenzscan zusätzlich zu einem Schicht-GRAPPA Referenzscan benötigt. Insbesondere für niedrige PAT-Faktoren wird dazu ein vollständig gesampelter EPI-Scan verwendet. Für größere in-plane PAT-Faktoren, also GRAPPA-Faktoren > 2, und für abzubildende Körperregionen, welche für Bewegungen anfällig sind oder typischerweise Bewegungen aufweisen, also beispielsweise eine Bauchregion, führt ein solcher EPI-Scan als Referenzscan oftmals zu Falt-Artefakten (englisch: folding artifacts) in den Magnetresonanzbildern. Dies ist darauf zurückzuführen, dass der EPI-Scan für größere Beschleunigungsfaktoren mittels Interleaving akquiriert werden muss. Abtastungen unterschiedlicher Segmente einer Schicht sind dadurch zumindest durch eine jeweilige Repetitionszeit TR zeitlich voneinander beabstandet. Dies führt wiederum dazu, dass die entsprechenden Segmente oftmals während unterschiedlicher Bewegungs- oder Atemzustände oder -phasen erfasst werden.

Als verhältnismäßig bewegungsrobuste Alternative kann ein GRE-basierter Referenzscan für eine in-plane GRAPPA-Kalibrierung verwendet werden. Dazu kann eine herkömmliche GRE-Sequenz nacheinander für jede Schicht ausgeführt werden. Entsprechende Referenzdaten für eine vollständige Schicht können dabei typischerweise in etwa 100 ms ausgelesen, also erfasst werden.

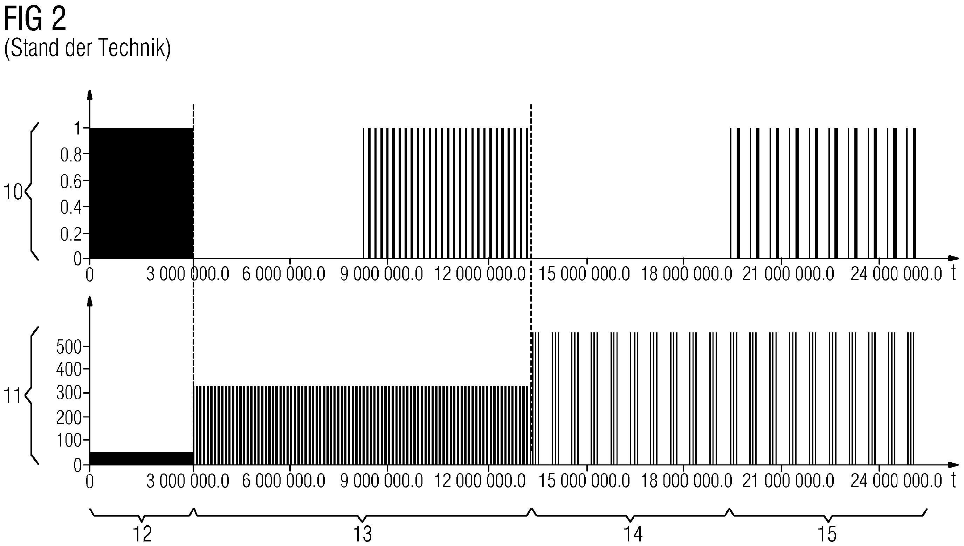

FIG 2 zeigt eine schematische Illustration eines Anfangsteiles eines Sequenzdiagramms für eine herkömmliche SMS-EPI-Datenakquisition für 20 Schichten. Entlang einer Zeitachse t aufgetragen sind hier ein ADC-Signal 10 und darunter parallel dazu ein RF-Signal 11. In einem ersten Zeitabschnitt 12 wird ein Einzelband GRE-Referenzscan für in-plane GRAPPA ausgeführt, welcher vorliegend für die 20 Schichten etwa 3 s beansprucht. Daran anschließend werden in einem Zeitabschnitt 13 zunächst ein einzelband dummy EPI-Scan für Schicht-GRAPPA und ein einzelband EPI-Referenzscan für Schicht-GRAPPA ausgeführt. Die hierfür benötigte zeitliche Länge des Zeitabschnitts 13 beträgt für die 20 Schichten im vorliegenden Beispiel etwa 10 s. In einem sich anschließenden Zeitabschnitt 14 wird zunächst ein multiband dummy EPI-Scan durchgeführt, um einen gleichmäßigen Gleichgewichtszustand (englisch: steady state) in allen Schichten herzustellen, um in den letztlich erzeugten Magnetresonanzbildern für alle Schichten gleiche Kontrastwerte zu erreichen. In einem sich daran anschließenden Zeitabschnitt 15 wird dann der eigentliche multiband Bildgebungs-EPI-Scan zum Akquirieren der Magnetresonanzdaten, welche letztlich in den Resonanzbildern dargestellt werden, ausgeführt. Es ist hier deutlich zu erkennen, dass der vorbereitende dummy EPI-Scan und der EPI-Referenzscan für das Schicht-GRAPPA-Verfahren im Zeitabschnitt 13 deutlich mehr Zeit beanspruchen als der GRE-basierte Referenzscan im Zeitabschnitt 12.

Der GRE-basierte Referenzscan im Zeitabschnitt 12 wird bisher jedoch nicht als Referenz für das Schicht-GRAPPA-Verfahren verwendet, da dann für die Korrektur der Geist-Artefakte benötigte Daten fehlen würden. Daher werden bisher der GRE-basierte Referenzscan im Zeitabschnitt 12 für eine in-plane GRAPPA-Kalibrierung, der dummy EPI-Scan für ein Präparieren eines gleichmäßigen Gleichgewichtszustandes und der EPI-Referenzscan im Zeitabschnitt 13 für eine Schicht-GRAPPA-Kalibrierung nacheinander ausgeführt. Beispielsweise für SMS-beschleunigte diffusionsbasierte Sequenzen mit einer Gesamtmesszeit von ungefähr einer Minute kann dies zu einer signifikanten Zeiteinbuße führen, welche die zeitlichen Vorteile einer gegenüber anderen Verfahren an sich schnelleren simultanen Mehrschicht-Akquisition (SMS)zumindest teilweise zunichtemachen kann.

Vor diesem Hintergrund wird vorliegend eine Lösung vorgeschlagen, welche eine Verwendung des GRE-basierten Referenzscans auch für die Schicht-GRAPPA-Kalibrierung erlaubt, wodurch vorteilhaft eine Akquisition der benötigten Referenzbeziehungsweise Korrekturdaten und damit die jeweilige Gesamtsequenz signifikant beschleunigt werden kann. Es wird hier vorgeschlagen, zusätzlich zu dem GRE-basierten Referenzscan einen dedizierten Phasenkorrekturscan zum Erfassen von Korrekturdaten für die Korrektur der Geist-Artefakte auszuführen.

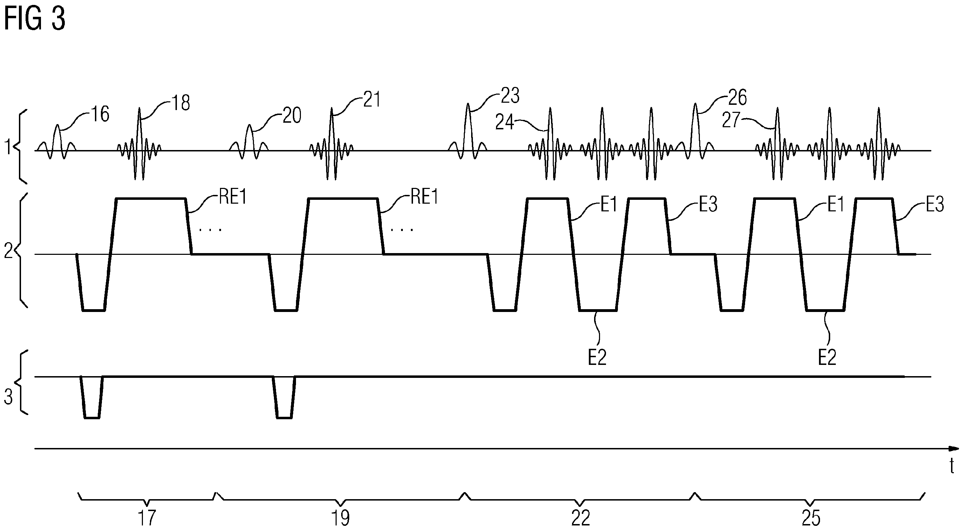

FIG 3 zeigt eine schematische und ausschnittsweise Illustration eines Sequenzdiagramms einer entsprechend verbesserten Sequenz für eine SMS-Bildgebung beziehungsweise - Datenakquisition in einer ersten Ausführungsform. Auch hier sind entlang einer Zeitachse t untereinander als erstes Signal 1 ein RF- beziehungsweise ADC-Signal, als zweites Signal 2 zugehörige Auslesegradienten (englisch: Readout gradient) und als drittes Signal 3 ein zugehöriges Phasenkodierungssignal beziehungsweise Phasenkodierungsgradient aufgetragen.

Vorliegend beginnt die Sequenz mit einem ersten Anregungspuls 16, welcher einen Anregungswinkel oder Flipwinkel α in einer ersten Schicht bewirkt. In einem Zeitabschnitt 17 wird zunächst ein erstes Referenzscanecho 18 akquiriert und entsprechende Referenzdaten in eine Referenzmatrix für die erste Schicht eingetragen. Beispielhaft ist hier ein entsprechender erster Readout-Gradient RE1 für das erste Referenzscanecho 18 dargestellt. Eine Referenzmatrix, also die Referenzdaten für eine Schicht, können typischerweise beispielsweise 20 bis 64 Referenzscanechos 18 umfassen, von denen der Übersichtlichkeit halber hier jeweils nur eines dargestellt ist. Nachdem die der GRE-Referenzscan für die erste Schicht abgeschlossen ist, wird in einem zweiten Zeitabschnitt 19 ein zweiter Anregungspuls 20 zum Anregen einer zweiten Schicht, also zum Erzeugen eines Anregungs- oder Flipwinkels, insbesondere des Flipwinkels a, in der zweiten Schicht, ausgegeben. Anschlie-βend wird ein zweites Referenzscanecho 21 akquiriert und entsprechende Referenzdaten für die zweite Schicht in die Referenzmatrix für die zweite Schicht eingetragen. Auch hier sind beispielhaft nur das erste Referenzscanecho 21 und ein zugehöriger erster Readout-Gradient RE1 für die zweite Schicht dargestellt. In der hier beispielhaft dargestellten Gesamtsequenz werden also die GRE-Referenzscans nacheinander für alle abzubildenden Schichten ausgeführt. Dies ist hier der Übersichtlichkeit halber lediglich für zwei Schichten dargestellt, kann aber ebenso für eine größere Anzahl von Schichten fortgesetzt werden.

Nachdem die GRE-Referenzscans für alle Schichten abgeschlossen sind, wird in einem anschließenden Zeitabschnitt 22 ein Geist-Korrektur- oder Phasenkorrekturscan für die erste Schicht ausgeführt. Dazu wird zunächst ein dritter Anregungspuls 23 erzeugt zum Einstellen eines Anregungs- oder Flipwinkel β in der ersten Schicht. Anschließend wird ein erster Phasenkorrekturechozug 24 erzeugt und die entsprechenden, zu den Auslesegradienten E1, E2, E3 korrespondierenden Navigator-Echos für die erste Schicht akquiriert. In entsprechender Weise werden dann in einem Zeitabschnitt 25 ein vierter Anregungspuls 26 und ein zweiter Phasenkorrekturechozug 27 erzeugt, um die entsprechenden, zu den Auslesegradienten E1, E2, E3 korrespondierenden Navigator-Echos für die zweite Schicht zu akquirieren. Es wird hier zum Akquirieren also ein vergleichsweise schnell ausführbarer 3-Echo-EPI-Phasenkorrekturscan nacheinander für alle Schichten ausgeführt. Mittels der dazu verwendeten Anregungspulse 23, 26 wird in den einzelnen Schichten jeweils der Anregungs- oder Flipwinkel β eingestellt.

Sowohl der für die GRE-Referenzscans verwendete Flipwinkel α als auch der für die Phasenkorrekturscans verwendete Flipwinkel β können dabei insbesondere kleiner als 90° sein, um Sättigungs- oder Vor-Sättigungseffekte vor einer sich an den Zeitabschnitt 25 anschließenden Bildgebungssequenz zu vermeiden oder zu minimieren, also um eine longitudinale Magnetisierung in den Schichten so weit wie möglich zu erhalten.

Die einzelband Navigator-Sequenzen zum Akquirieren der Phasenkorrekturdaten in den Zeitabschnitten 22, 25 können dabei für jede einzelne Schicht in beispielsweise 5 ms ausgeführt werden, sodass eine zusätzlich zum Akquirieren des entsprechenden Korrekturdatensatzes insbesondere im Vergleich zu den in dem in FIG 2 dargestellten Sequenzdiagramm in Zeitabschnitt 13 ausgeführten EPI-Scans sehr klein ist.

Anders als in dem Sequenzdiagramm in FIG 3 dargestellt können die Phasenkorrekturscans ebenso zeitlich vor den GRE-Referenzscans für alle Schichten ausgeführt werden.

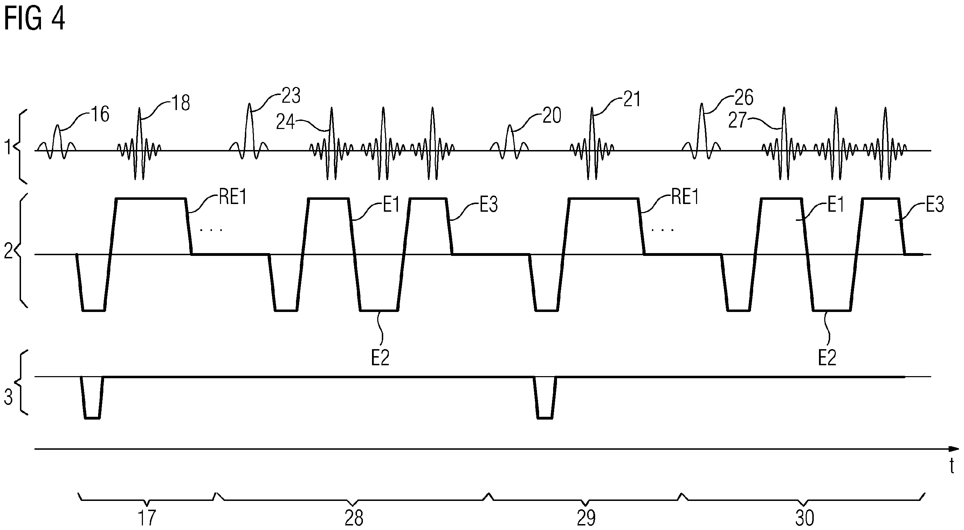

FIG 4 zeigt eine schematische ausschnittweise Illustration eines Sequenzdiagramms einer verbesserten Sequenz für eine SMS-Magnetresonanzbildgebung in einer zweiten Ausführungsform. Die einzelnen Komponenten entsprechen dabei im Wesentlichen den bereits in Zusammenhang mit FIG 3 beschriebenen Komponenten, sodass hier nur kurz die Unterschiede erläutert werden. Für die in FIG 4 angedeutete Sequenz wird eine Interleaving-Methode für die GRE-Referenzscans und die Phasenkorrekturscans verwendet.

Auch hier wird zunächst in dem Zeitabschnitt 17 der GRE-Referenzscan für die erste Schicht ausgeführt. Daran anschließend wird jedoch in einem Zeitabschnitt 28 zunächst der Phasenkorrekturscan für die erste Schicht ausgeführt. In diesem Sinne entspricht der Zeitabschnitt 28 funktional also dem Zeitabschnitt 25 aus FIG 3. Anschließend wird in einem Zeitabschnitt 29 der GRE-Referenzscan für die zweite Schicht und daran anschließend in einem Zeitabschnitt 30 der Phasenkorrekturscan für diese zweite Schicht ausgeführt. In diesem Sinne entspricht also der Zeitabschnitt 29 funktional dem Zeitabschnitt 19 aus FIG 3 und der Zeitabschnitt 30 dem Zeitabschnitt 25 aus FIG 3. Auch hier können die GRE-Referenzscans und die Phasenkorrekturscans zeitlich vertauscht werden, sodass also beispielsweise zunächst der Phasenkorrekturscan für die erste Schicht, dann der GRE-Referenzscan für die erste Schicht - oder umgekehrt - und dann die entsprechenden Scans für die zweite Schicht durchgeführt werden können.

In der in FIG 4 dargestellten Sequenz können die jeweils zwischen einem GRE-Referenzscan und einem Phasenkorrekturscan für eine Schicht vorgesehenen Anregungspulse, hier also der dritter Anregungspuls 23 und der vierte Anregungspuls 26 je nach Auslegung oder Eignung einer jeweils verwendeten Magnetresonanzanlage weggelassen werden. Es kann dann jeweils für eine Schicht nur ein Anregungspuls vorgesehen sein, nach welchem dann für diese Schicht ohne weiteren Anregungspuls sowohl der GRE-Referenzscan als auch der Phasenkorrekturscan ausgeführt werden.

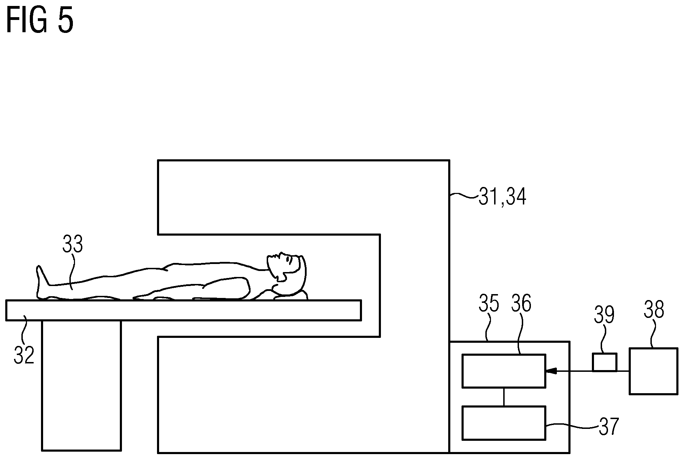

FIG 5 zeigt eine schematische Darstellung einer Magnetresonanzanlage 31 mit einer Patientenliege 32, auf welcher vorliegend ein Patient 33 angeordnet ist. Die Magnetresonanzanlage 31 umfasst vorliegend eine hier nicht im Detail dargestellte Aufnahmeeinrichtung zum Erfassen oder Aufnehmen von Magnetresonanzdaten. Die Aufnahmeeinrichtung 34 kann beispielsweise entsprechende Magnetspulen, einen Leistungsverstärker, einen Pulsformer, einen Signalprozessor und/oder weitere elektrische oder elektronische Komponenten umfassen. Weiterhin ist hier eine Datenverarbeitungseinrichtung 35 angedeutet, welche einen Prozessor 36 und einen mit diesem verbundenen Datenträger 37 umfasst. Die Datenverarbeitungseinrichtung 35 kann Teil der Magnetresonanzanlage, insbesondere in diese integriert, sein. Ebenso kann die Datenverarbeitungseinrichtung 35 jedoch eine separate Einrichtung sein, welche beispielsweise von der Magnetresonanzanlage 31 oder von der Aufnahmeeinrichtung 34 separat, beispielsweise onpremise in einem lokalen Rechenzentrum, angeordnet sein kann. Ebenso kann die Datenverarbeitungseinrichtung 35 beispielsweise ein entfernt angeordnetes Rechenzentrum, ein Cloud-Server oder dergleichen sein. Die Magnetresonanzanlage 31 und die Datenverarbeitungseinrichtung 35 können dann beispielsweise mittels entsprechender Datenleitungen oder Datenverbindungen zu einem Magnetresonanzsystem miteinander verbunden sein.

Auf dem Datenträger 37 ist vorliegend ein Computerprogramm oder Computerprogrammprodukt, also ein mittels des Prozessors 36 ausführbarer Programmcode, gespeichert, welcher Verfahrensschritte eines Verfahrens zum Erzeugen von Magnetresonanzbildern mittels der Magnetresonanzanlage 31 kodiert oder repräsentiert, also entsprechende Steuer- und/oder Rechenbefehle umfasst. Durch Ausführen des auf dem Datenträger 37 gespeicherten Computerprogramms mittels des Prozessors 36 wird also die Magnetresonanzanlage 31 dazu veranlasst, gemäß der insbesondere im Zusammenhang mit FIG 3 und FIG 4 beschriebenen Weise einen Referenzdatensatz, einen Korrekturdatensatz und einen Messdatensatz aufzunehmen. Der Referenzdatensatz wird dabei durch Ausführen eines Referenzscans des Patienten 33 für jede abzubildende Schicht mittels einer GRE-Sequenz oder einer RARE-Sequenz aufgenommen. Der Korrekturdatensatz wird durch Ausführen eines Phasenkorrekturscans des Patienten 33 für jede abzubildende Schicht mittels einer nicht-phasenkodierenden EPI-Sequenz aufgenommen. Der Messdatensatz wird mittels einer SMS-Sequenz, insbesondere einer SMS-EPI-Sequenz, aufgenommen. Mittels der Datenverarbeitungseinrichtung 35 werden aus dem Referenzdatensatz dann schichtspezifische GRAPPA-Kerne für die einzelnen Schichten bestimmt. Weiter werden mittels der Datenverarbeitungseinrichtung 35 Magnetresonanzbilder des Patienten 33 aus dem Messdatensatz durch ein Schicht-GRAPPA-Verfahren erzeugt. Dabei werden zu unterschiedlichen der abgebildeten Schichten gehörende Daten des Messdatensatzes mittels der schichtspezifischen GRAPPA-Kerne voneinander separiert und Nyquist-N/2-Geist-Artefakte in dem Messdatensatz mittels des Korrekturdatensatzes korrigiert.

Zusätzlich oder alternativ zu dem Speichern des Computerprogramms auf dem Datenträger 37 kann das entsprechende Computerprogramm ebenso beispielsweise auf einem externen Datenspeicher 38 gespeichert und dann als ein Datenträgersignal 39 an die Datenverarbeitungseinrichtung 35 übermittelt oder übertragen werden.

Insgesamt zeigen die beschriebenen Beispiele, wie durch Akquirieren von Phasenkorrekturdaten mittels eines Phasenkorrektur-EPI-Scans und Akquirieren von Referenzdaten für eine Schicht-GRAPPA-Kalibrierung eine beschleunigte Datenakquisition für eine SMS-Magnetresonanzbildgebung realisiert werden kann.

## Patentansprüche

1. Verfahren zum Erzeugen von Magnetresonanzbildern eines Zielobjekts (33) mit den Verfahrensschritten
- Aufnehmen eines separaten Referenzdatensatzes für jede abzubildende Schicht des Zielobjekts (33) mittels einer Magnetresonanzanlage (31) durch Ausführen eines jeweiligen Referenzscans (16, 18, 20, 21, RE1) des Zielobjekts (33) für jede abzubildende Schicht des Zielobjekts (33) mittels einer GRE-Sequenz oder einer RARE-Sequenz,
- Aufnehmen eines separaten Korrekturdatensatzes für jede abzubildende Schicht des Zielobjekts (33) mittels der Magnetresonanzanlage (31) durch Ausführen eines jeweiligen Phasenkorrekturscans (23, 24, 26, 27, E1, E2, E3) des Zielobjekts (33) für jede abzubildende Schicht des Zielobjekts (33) mittels einer nicht-phasenkodierenden EPI-Sequenz,
- Aufnehmen eines Messdatensatzes des Zielobjekts (33) mittels der Magnetresonanzanlage (31) mittels einer SMS-Sequenz (14, 15),
- Bestimmen von schichtspezifischen GRAPPA-Kernen aus dem Referenzdatensatz, und
- Erzeugen der Magnetresonanzbilder des Zielobjekts (33) aus dem Messdatensatz durch ein Schicht-GRAPPA-Verfahren mittels einer Datenverarbeitungseinrichtung (35), wobei
- zu unterschiedlichen der abgebildeten Schichten gehörende Daten des Messdatensatzes mittels der schichtspezifischen GRAPPA-Kerne voneinander separiert werden, und
- Nyquist-N/2-Geist-Artefakte in dem Messdatensatz mittels des Korrekturdatensatzes korrigiert werden.

2. Verfahren nach Anspruch 1, **dadurch gekennzeichnet, dass** die Referenzscans (16, 18, 20, 21, RE1) für alle Schichten aufeinanderfolgend ausgeführt werden und die Phasenkorrekturscans (23, 24, 26, 27, E1, E2, E3) für alle Schichten ebenfalls aufeinanderfolgend ausgeführt werden, wobei alle der Phasenkorrekturscans (23, 24, 26, 27, E1, E2, E3) ausgeführt werden nachdem alle der Referenzscans (16, 18, 20, 21, RE1) ausgeführt worden sind oder bevor alle der Referenzscans (16, 18, 20, 21, RE1) ausgeführt werden.

3. Verfahren nach Anspruch 1, **dadurch gekennzeichnet, dass** die Referenzscans (16, 18, 20, 21, RE1) und die Phasenkorrekturscans (23, 24, 26, 27, E1, E2, E3) in verschachtelter Reihenfolge ausgeführt werden, wobei für jede der abzubildenden Schichten der jeweilige Referenzscan (16, 18, 20, 21, RE1) und der jeweilige Phasenkorrekturscan (23, 24, 26, 27, E1, E2, E3) ausgeführt werden, bevor die jeweiligen Referenzscans (16, 18, 20, 21, RE1) und Phasenkorrekturscans (23, 24, 26, 27, E1, E2, E3) für die jeweils nächste der abzubildenden Schichten ausgeführt werden.

4. Verfahren nach Anspruch 3, **dadurch gekennzeichnet, dass** für jeweils eine der Schichten nur ein Anregungspuls (16, 20; 23, 26) erzeugt wird und nach diesem der jeweilige Referenzscan (16, 18, 20, 21, RE1) und der jeweilige Phasenkorrekturscan (23, 24, 26, 27, E1, E2, E3) für die jeweilige Schicht ausgeführt werden, ohne dass zwischen dem Referenzscan (16, 18, 20, 21, RE1) und dem Phasenkorrekturscan (23, 24, 26, 27, E1, E2, E3) für die jeweilige Schicht ein zweiter Anregungspuls (23, 26; 16, 20) für diese Schicht erzeugt wird.

5. Verfahren nach einem der vorhergehenden Ansprüche, **dadurch gekennzeichnet, dass** für den Korrekturdatensatz jeweils wenigstens drei Echos (E1, E2, E3) aus jedem der Phasenkorrekturscans (23, 24, 26, 27, E1, E2, E3) verwendet werden, wobei jeweils ein Mittelwert aus dem ersten Echo (E1) und dem dritten Echo (E3) gebildet wird, um eine zeitliche Konsistenz mit dem jeweiligen zweiten Echo (E2) zu erreichen.

6. Verfahren nach einem der vorhergehenden Ansprüche, **dadurch gekennzeichnet, dass** für die Referenzscans und die Phasenkorrekturscans (16, 18, 20, 21, RE1) Flipwinkel von weniger als 90° verwendet werden.

7. Verfahren nach einem der vorhergehenden Ansprüche, **dadurch gekennzeichnet, dass** der Messdatensatz mittels einer multiband SMS-EPI-Sequenz aufgenommen wird, insbesondere für eine diffusionsbasierte Abbildung des Zielobjekts (33).

8. Verfahren nach einem der vorhergehenden Ansprüche, **dadurch gekennzeichnet, dass** der Referenzdatensatz mit geringerer Auflösung aufgenommen wird als der Messdatensatz.

9. Verfahren nach einem der vorhergehenden Ansprüche, **dadurch gekennzeichnet, dass** Sensitivitätsvariationen zwischen verschiedenen für das Aufnehmen des Messdatensatzes parallel verwendeten Empfängerspulen bestimmt und bei dem Erzeugen der Magnetresonanzbilder berücksichtigt werden.

10. Verfahren nach einem der vorhergehenden Ansprüche, **dadurch gekennzeichnet, dass** zusätzlich ein in-plane-GRAPPA-Verfahren verwendet wird und der Referenzdatensatz auch zum Kalibrieren der GRAPPA-Kerne für das in-plane-GRAPPA-Verfahren verwendet wird.

11. Computerprogramm, umfassend Befehle, die bei einer Ausführung des Computerprogramms durch eine Computereinrichtung (35) einer Magnetresonanzanlage (31) die Magnetresonanzanlage (31) dazu veranlassen, ein Verfahren nach einem der vorhergehenden Ansprüche auszuführen.

12. Datenträgersignal, welches das Computerprogramm nach Anspruch 11 überträgt.

13. Computerlesbarer Datenträger (37, 38), auf welchem ein Computerprogramm nach Anspruch 11 gespeichert ist.

14. Magnetresonanzanlage (31) mit einer Aufnahmeeinrichtung (34) zum Aufnahmen von Magnetresonanzdaten und mit einer Datenverarbeitungseinrichtung (35) zum Verarbeiten der aufgenommenen Magnetresonanzdaten, wobei die Datenverarbeitungseinrichtung (35) einen computerlesbaren Datenträger (37, 38) nach Anspruch 13 und eine mit diesem verbundene Prozessoreinrichtung (36) umfasst, welche zum Ausführen des auf dem computerlesbaren Datenträger (37, 38) gespeicherten Computerprogramms zum Ausführen eines Verfahrens nach einem der Ansprüche 1 bis 10 eingerichtet ist.

## Claims

1. Method for creating magnetic resonance images of a target object (33) with the method steps
- Recording of a separate reference dataset for each slice of the target object (33) to be imaged by means of a magnetic resonance system (31) by carrying out a respective reference scan (16, 18, 20, 21, RE1) of the target object (33) by means of a GRE sequence or a RARE sequence for each slice of the target object (33) to be imaged,
- Recording of a separate correction dataset for each slice of the target object (33) to be imaged by means of the magnetic resonance system (31) by carrying out a respective phase correction scan (23, 24, 26, 27, E1, E2, E3) of the target object (33) by means of a non-phase-encoding EPI sequence for each slice of the target object (33) to be imaged,
- Recording of a measurement dataset of the target object (33) by means of the magnetic resonance system (31) by means of an SMS sequence (14, 15),
- Determination of slice-specific GRAPPA kernels from the reference dataset, and
- Creation of the magnetic resonance images of the target object (33) from the measurement dataset by a slice GRAPPA method by means of a data processing device (35), wherein
- Data of the measurement dataset belonging to different slices of the slices imaged is separated from one another by means of the slice-specific GRAPPA kernels, and
- Nyquist-N/2 ghost artifacts in the measurement dataset are corrected by means of the correction dataset.

2. Method according to claim 1, **characterised in that** the reference scans (16, 18, 20, 21, RE1) for all slices are carried one after the other and the phase correction scans (23, 24, 26, 27, E1, E2, E3) for all slices are likewise carried out one after the other, wherein all of the phase correction scans (23, 24, 26, 27, E1, E2, E3) are carried out after all of the reference scans (16, 18, 20, 21, RE1) have been carried out or before all the reference scans (16, 18, 20, 21, RE1) are carried out.

3. Method according to claim 1, **characterised in that** the reference scans (16, 18, 20, 21, RE1) and the phase correction scans (23, 24, 26, 27, E1, E2, E3) are carried out in a nested order, wherein for each of the slices to be imaged, the respective reference scan (16, 18, 20, 21, RE1) and the respective phase correction scan (23, 24, 26, 27, E1, E2, E3) are carried out before the respective reference scans (16, 18, 20, 21, RE1) and phase correction scans (23, 24, 26, 27, E1, E2, E3) for the next of the slices to be imaged in each case.

4. Method according to claim 3, **characterised in that** only one excitation pulse (16, 20; 23, 26) is created for each one of the slices and after this the respective reference scan (16, 18, 20, 21, RE1) and the respective phase correction scan (23, 24, 26, 27, E1, E2, E3) is carried out for the respective slice, without a second excitation pulse (23, 26; 16, 20) being created for this slice between the reference scan (16, 18, 20, 21, RE1) and the phase correction scan (23, 24, 26, 27, E1, E2, E3) for the respective slice.

5. Method according to one of the preceding claims, **characterised in that** at least three echoes (E1, E2, E3) from each of the phase correction scans (23, 24, 26, 27, E1, E2, E3) are used for the correction dataset in each case, wherein an average value is formed from the first echo (E1) and the third echo (E3) in each case, in order to obtain a temporal consistency with the respective second echo (E2).

6. Method according to one of the preceding claims, **characterised in that** flip angles of less than 90° are used for the reference scans and the phase correction scans (16, 18, 20, 21, RE1).

7. Method according to one of the preceding claims, **characterised in that** the measurement dataset is recorded by means of a multiband SMS-EPI sequence, in particular for a diffusion-based imaging of the target object (33).

8. Method according to one of the preceding claims, **characterised in that** the reference dataset is recorded with lower resolution than the measurement dataset.

9. Method according to one of the preceding claims, **characterised in that** sensitivity variations between different receiver coils used in parallel for the recording of the measurement dataset are determined and taken into account during the creation of the magnetic resonance images.

10. Method according to one of the preceding claims, **characterised in that** in addition an in-plane GRAPPA method is used and the reference dataset is also used for calibrating the GRAPPA kernels for the in-plane GRAPPA method.

11. Computer program, comprising commands that, on execution of the computer program by a computer device (35) of a magnetic resonance system (31), cause the magnetic resonance system (31) to carry out a method according to one of the preceding claims.

12. Data medium signal, which transmits the computer program according to claim 11.

13. Computer-readable data medium (37, 38) on which a computer program according to claim 11 is stored.

14. Magnetic resonance system (31) with a recording device (34) for recording magnetic resonance data and with a data processing device (35) for processing the recorded magnetic resonance data, wherein the data processing device (35) comprises a computer-readable data medium (37, 38) according to claim 13 and comprises a processor device (36) connected to said medium, which is configured to carry out the computer program stored on the computer-readable data medium (37, 38) for carrying out a method according to one of claims 1 to 10.

## Revendications

1. Procédé de production d'images par résonnance magnétique d'un objet (33) cible comprenant les stades de procédé
- enregistrement d'un ensemble distinct de données de référence pour chaque tranche à représenter de l'objet (33) cible au moyen d'une installation (31) par résonnance magnétique, en effectuant un scan (16, 18, 20, 21, RE1) respectif de référence de l'objet (33) cible, pour chaque tranche à reproduire de l'objet (33) cible, au moyen d'une séquence GRE ou d'une séquence RARE,
- enregistrement d'un ensemble distinct de données de correction, pour chaque tranche à représenter de l'objet (33) cible au moyen de l'installation (31) par résonnance magnétique, en effectuant un scan (23, 24, 26, 27, E1, E2, E3) respectif de correction de phase de l'objet (33) cible, pour chaque tranche à représenter de l'objet (33) cible, au moyen d'une séquence EPI non codée en phase,
- enregistrement d'un ensemble de données de mesure de l'objet (33) cible au moyen de l'installation (31) par résonnance magnétique, au moyen d'une séquence SMS (14, 15),
- détermination de noyaux GRAPPA spécifique à une tranche à partir de l'ensemble de données de référence, et
- production des images par résonnance magnétique de l'objet (33) cible à partir de l'ensemble de données de mesure, par un procédé GRAPPA de tranche au moyen d'un dispositif (35) de traitement de données, dans lequel
- on sépare les unes des autres, au moyen des noyaux GRAPPA spécifiques à une tranche, des données de l'ensemble de données de mesure appartenant à des tranches différentes des tranches représentées, et
- on corrige des artefacts fantômes N/2 de Nyquist dans l'ensemble de données de mesure au moyen de l'ensemble de données de correction.

2. Procédé suivant la revendication 1, **caractérisé en ce que** l'on réalise les scans (16, 18, 20, 21, RE1) de référence successivement pour toutes les tranches et on réalise les scans (23, 24, 26, 27, E1, E2, E3) également successivement pour toutes les tranches, dans lequel on réalise tous les scans (23, 24, 26, 27, E1, E2, E3) de correction de phase, après avoir réalisé tous les scans (16, 18, 20, 21, RE1) de référence ou avant de réaliser tous les scans (16, 18, 20, 21, RE1) de référence.

3. Procédé suivant la revendication 1, **caractérisé en ce que** l'on réalise les scans (16, 18, 20, 21, RE1) de référence et les scans (23, 24, 26, 27, E1, E2, E3) de correction de phase en séquence imbriquée, dans lequel on réalise pour chacune des tranches à représenter le scan (16, 18, 20, 21, RE1) respectif de référence et le scan (23, 24, 26, 27, E1, E2, E3) respectif de correction de phase, avant de réaliser le scan (16, 18, 20, 21, RE1) respectif de référence et le scan (23, 24, 26, 27, E1, E2, E3) respectif de correction de phase pour les suivantes des tranches à représenter.

4. Procédé suivant la revendication 3, **caractérisé en ce que** l'on produit pour, respectivement une des tranches seulement une impulsion (16, 20 ; 23, 26) d'excitation et après celle-ci on réalise le scan (16, 18, 20, 21, RE1) respectif de référence et le scan (23, 24, 26, 27, E1, E2, E3) respectif de correction de phase pour la tranche respective, sans produire entre le scan (16, 18, 20, 21, RE1) de référence et le scan (23, 24, 26, 27, E1, E2, E3) de correction de phase pour la tranche respective une deuxième impulsion (23, 26 ; 16, 20) d'excitation pour cette tranche.

5. Procédé suivant l'une des revendications précédentes, **caractérisé en ce que** l'on utilise pour l'ensemble de données de correction respectivement au moins trois échos (E1, E2, E3) à partir de chacun des scans (23, 24, 26, 27, E1, E2, E3) de correction de phase, dans lequel on forme respectivement une valeur moyenne à partir du premier écho (E1) et du troisième écho (E3), pour obtenir une cohérence temporelle avec le deuxième écho (E2) respectif.

6. Procédé suivant l'une des revendications précédentes, **caractérisé en ce que** l'on utilise des angles de flip de moins de 90° pour les scans de référence et pour les scans (16, 18, 20, 21, RE1) de correction de phase.

7. Procédé suivant l'une des revendications précédentes, **caractérisé en ce que** l'on enregistre l'ensemble de données de mesure au moyen d'une séquence SMS-EPI multi-bande, notamment pour une représentation de l'objet (33) cible reposant sur une diffusion.

8. Procédé suivant l'une des revendications précédentes, **caractérisé en ce que** l'on enregistre l'ensemble de données de référence à une résolution plus petite que l'ensemble de données de mesure.

9. Procédé suivant l'une des revendications précédentes, **caractérisé en ce que** l'on détermine des variations de sensibilité entre diverses bobines réceptrices utilisées en parallèle pour l'enregistrement de l'ensemble de données de mesure et on en tient compte lors de la production des images par résonnance magnétique.

10. Procédé suivant l'une des revendications précédentes, **caractérisé en ce que** l'on utilise en plus un procédé GRAPPA-in-plane et on utilise l'ensemble de données de référence également pour l'étalonnage des noyaux GRAPPA pour le procédé GRAPPA-in-plane.

11. Programme d'ordinateur, comprenant des instructions, qui lorsque le programme d'ordinateur est réalisé par un dispositif (35) informatique d'une installation (31) par résonnance magnétique, font que l'installation (31) par résonnance magnétique effectue un procédé suivant l'une des revendications précédentes.

12. Signal de support de données, qui transmet le programme d'ordinateur suivant la revendication 11.

13. Support (37, 38) de données, déchiffrables par ordinateur, sur lequel est mémorisé un programme d'ordinateur suivant la revendication 11.

14. Installation (31) par résonnance magnétique comprenant un dispositif (34) d'enregistrement pour l'enregistrement de données de résonnance magnétique et comprenant un dispositif (35) de traitement de données pour le traitement des données de résonnance magnétique enregistrées, dans laquelle le dispositif (35) de traitement de données comprend un support (37, 38) de données déchiffrable par ordinateur suivant la revendication 13 et un dispositif (36) de processeur relié à celui-ci, qui est, pour l'exécution d'un procédé suivant l'une des revendications 1 à 10, conçu pour l'exécution du programme d'ordinateur mis en mémoire sur le support (37, 38) de données déchiffrable par ordinateur suivant un procédé suivant l'une des revendications 1 à 10.
